(19) Europäisches Patentamt
European Patent Office
Office européen des brevets

(11) Veröffentlichungsnummer: **0 429 453 B1**

(12) # EUROPÄISCHE PATENTSCHRIFT

(45) Veröffentlichungstag der Patentschrift: **09.06.93**

(51) Int. Cl.[5]: **C07C 409/24**

(21) Anmeldenummer: **89903740.2**

(22) Anmeldetag: **06.03.89**

(86) Internationale Anmeldenummer:
**PCT/EP89/00228**

(87) Internationale Veröffentlichungsnummer:
**WO 89/08642 (21.09.89 89/23)**

(54) **DIISOPROPYLBENZOL-BISPERNEOALKANOATE.**

(30) Priorität: **08.03.88 DE 3807564**

(43) Veröffentlichungstag der Anmeldung:
**05.06.91 Patentblatt 91/23**

(45) Bekanntmachung des Hinweises auf die Patenterteilung:
**09.06.93 Patentblatt 93/23**

(84) Benannte Vertragsstaaten:
**BE DE FR GB IT NL**

(56) Entgegenhaltungen:
**FR-A- 1 460 360**
**FR-A- 2 159 269**
**FR-A- 2 376 844**

(73) Patentinhaber: **Peroxid-Chemie GmbH**
**Dr.-Gustav-Adolf-Strasse 3**
**W-8023 Höllriegelskreuth(DE)**

(72) Erfinder: **DORN, Maximilian**
**Gistlstrasse 100 A**
**W-8023 Pullach(DE)**
Erfinder: **HÄGEL, Eberhard**
**Eichendorffstrasse 26 a**
**W-8021 Icking(DE)**
Erfinder: **ZEISS, Werner**
**Moosstrasse 15**
**W-8038 Gröbenzell(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08**
**20**
**W-8000 München 86 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Diisopropylbenzol-bisperneoalkanoate und ihre Verwendung als Polymerisationsinitiatoren.

Es ist bekannt, daß Perester zur Durchführung von chemischen Reaktionen, die durch freie Radikale initiiert werden, insbesondere von Radikalkettenpolymerisationen und -mischpolymerisationen, geeignet sind. Ethylenisch ungesättigte, polymerisierbare Monomere, wie Ethylen, Styrol, (Meth)acrylsäureester, Vinylchlorid, Vinylacetat und andere Vinylester oder Gemische davon, wie z.B. Ethylen und Vinylacetat, Styrol und Acrylnitril, Vinylchlorid und Vinylacetat, können unter dem Einfluß von Verbindungen, die leicht freie Radikale bilden, polymerisiert oder copolymerisiert werden; ebenso kann zum Beispiel auch die Copolymerisation von "ungesättigten Polyesterharzen", d.h. von Gemischen aus ungesättigten Polyestern und einem oder mehreren ethylenisch ungesättigten Monomeren, wie z.B. Styrol, unter dem Einfluß von freie Radikale bildenden Verbindungen bewirkt werden.

Aus der DE-PS 19 22 183 sind tert.-Alkylperester von tertiären Hydroperoxiden als Initiatoren für die Polymerisation von Monomeren, wie Styrol, Vinylchlorid, Vinylacetat und Ethylen bekannt; das tertiäre α-Kohlenstoffatom der für die Herstellung der Perester verwendeten organischen Säure enthält in diesen Verbindungen nicht mehr als zwei Methylgruppen und vorzugsweise keine Methylgruppe.

Aus der FR-A-2376844 ist eine Diperoxyester Zusammensetzung bekannt, die aus mindestens drei verschiedenen Diperoxyester-Komponenten besteht, wobei zwei der Komponenten immer symmetrisch sind, und die dritte Komponente ein asymmetrisches Hybrid aus den beiden anderen Peroxyestern ist. In dieser Diperoxyester-Zusammensetzung sind die unsymmetrischen Komponenten jeweils in deutlich verschiedenen Temperaturbereichen als Initiator wirksam, wobei "deutlich verschiedene Temperaturbereiche" bedeutet, daß die symmetrischen Komponenten und die unsymmetrische Komponente des Gemisches aus Verbindungen erzeugt werden, die Nieder-, Mittel- oder Hochtemperatur-Peroxyester bildende Carbonylverbindungen darstellen.

Die Diperoxyester-Zusammensetzung wird als gut geeignete stufenweise wirksam werdende Initiatorkombination für die Polymerisation von ethylenisch ungesättigten Monomeren sowie als Härtungsmittel für ungesättigte Polyesterharze beschrieben.

In der DE-OS 22 49 960 werden Perester beschrieben, die sich vom α-Cumolhydroperoxid und solchen organischen Säuren ableiten, in welchen das α-Kohlenstoffatom sekundär ist und zwei Alkylgruppen aufweist, die zusammen 3 bis 10 Kohlenstoffatome enthalten oder in welchen dieses α-Kohlenstoffatom in einer Cyclohexylgruppe vereinigt ist, die gegebenenfalls substituiert sein kann. Gegenstand der vorliegenden Erfindung sind Diisopropylbenzol-bisperneoalkanoate der allgemeinen Formel I

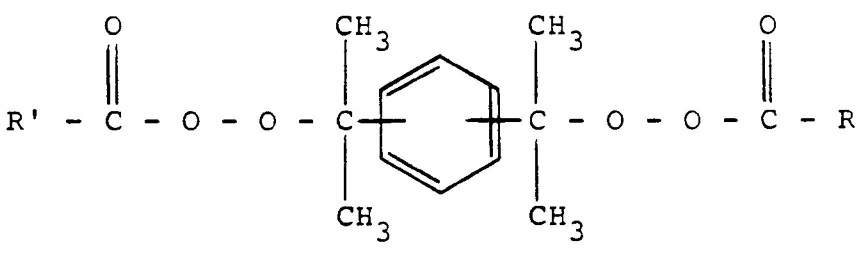

(I)

in der R und R' eine $-CR_1R_2R_3$-Gruppe bedeuten, worin $R_1$, $R_2$ und $R_3$ eine Alkylgruppe bedeutet und $R_1$, $R_2$ und $R_3$ zusammen 3 bis 11, vorzugsweise 5 bis 8 Kohlenstoffatome und insbesondere 8 Kohlenstoffatome enthalten. R und R' können verschieden sein und sind vorzugsweise gleich. Insbesondere bedeutet R und R' einen Neononylrest.

Im Diisopropylbenzol-Rest stehen die Isopropylgruppen in m- und/oder p-Stellung zueinander; es handelt sich also um die 1,3- oder 1,4-Isomeren, oder um deren Gemische.

Eine besonders bevorzugte erfindungsgemäße Verbindung ist das Diisopropylbenzol-Bis-(perneodecanoat).

Die neuen Verbindungen der allgemeinen Formel I können analog an sich bekannter Methoden hergestellt werden, wie sie zum Beispiel für ähnliche Verbindungen aus der DE-PS 19 22 183 und der DE-OS 22 49 960 bekannt sind. Vorteilhafterweise werden sie durch Umsetzung äquivalenter Mengen von Bis-(hydroperoxy-isopropyl)-benzol mit einer Verbindung der Formel R-COCl und/oder R'-COCl hergestellt; vorzugsweise sind R und R' gleich.

Die Reaktionsbedingungen entsprechen dabei in der Regel den für eine solche Umsetzung üblichen Bedingungen (vgl. z.B. DE-PS 19 22 183; DE-OS 22 49 960). Die Reaktionstemperatur liegt im allgemeinen zwischen -5 und 25 °C; die Reaktionen können in Wasser durchgeführt werden. Hierbei kann gegebenenfalls ein Emulgator anwesend sein. Die Reaktion kann aber auch in einem Gemisch aus Petrolether und Wasser stattfinden; im letzteren Fall wird zur Isolierung des Peresters die organische Schicht mit Wasser gewaschen, anschließend getrocknet und der Petrolether dann abdestilliert.

Zur Herstellung der erfindungsgemäßen Perester der Formel I geht man vorzugsweise von den isomeren Gemischen der entsprechenden Neoalkansäuren, bzw. von den davon abgeleiteten Säurechloriden aus. Die Isomerenverteilung kann dabei der in Tabelle I der DE-PS 19 22 183 für solche Säuren angegebenen Verteilung entsprechen.

Das als Ausgangsprodukt verwendete Bis-(hydroperoxyisopropyl)-benzol ist das m- oder p-Bis-(hydroperoxyisopropyl)-benzol; es können aber auch Isomerengemische von m- und p-Verbindungen eingesetzt werden. Diese Verbindungen sind bekannt und können nach an sich bekannten Methoden hergestellt werden (vgl. z.B. DE-OS 29 42 069, GB-PS 13 14 069, J.Chem.Soc. Japan, 81, 582 (1960)).

Es wurde gefunden, daß die erfindungsgemäßen Verbindungen, und insbesondere das Diisopropylbenzol-bisperneodecanoat, sich sehr gut als Initiator für die Polymerisation und die Copolymerisation ethylenisch ungesättigter Monomerer eignen; in vieler Hinsicht zeigen sie dabei gegenüber bekannten und hierfür üblichen Initiatoren, wie z.B. ACSP (Acetyl-cyclohexansulfonyl-peroxid) und CUPND (Cumylperoxyneodecanoat) vorteilhaftere Eigenschalten. So hat sich z.B. gezeigt, daß die erfindungsgemäßen Verbindungen, und insbesondere das Diisopropylbenzol-bisperneodecanoat, bei der Vinylchlorid-Polymerisation im Vergleich zu ACSP und CUPND gleichwertig sind oder sogar schneller zerfallen, wodurch eine kürzere Polymerisationszeit bei der Herstellung von Polyvinylchlorid ermöglicht wird; es hat sich auch gezeigt, daß z.B. bei der Ethylen-Polymerisation bei tieferen Temperaturen polymerisiert werden kann und im Vergleich zu den üblichen Initiatoren insbesondere Copolymere mit höherem Molekulargewicht erhalten werden können. Außerdem besitzen die Zerfallsprodukte der beanspruchten Perester keinen unangenehmen Geruch wie z.B. bei CUPND und zusätzlich auch keinen korrosiven Charakter wie z.B. bei ACSP.

Gegenstand der Erfindung ist deshalb auch die Verwendung der erfindungsgemäßen Verbindungen der Formel I und insbesondere der bevorzugt genannten Verbindungen der Formel I, als Initiatoren von Radikalkettenpolymerisationen oder -mischpolymerisationen, und insbesondere zur Polymerisation von Vinylchlorid und von Ethylen.

Die nachfolgenden Beispiele sollen die Erfindung näher erläutern, ohne sie darauf zu beschränken.

Beispiel 1

In 481 g (3,0 Mol) 35 % Kalilauge werden unter Rühren 227 g (0,5 Mol) p-Bis-(hydroperoxy-isopropyl)-benzol (50 % wasserfeucht) gelöst und nach Zugabe von 135 g Isododecan werden 192 g (1,0 Mol) Neodecansäurechlorid bei 25 °C zugetropft. Anschließend wird bei 25 °C noch 1 Stunde nachgerührt. Die wäßrige Phase wird abgetrennt, die organische Phase wird mit 300 ml 15 % Natronlauge, dann dreimal mit je 300 ml 10 % Natriumsulfat-Lösung gewaschen. Nach Trocknen mit Natriumsulfat und Filtrieren erhält man 330 g einer viskosen Flüssigkeit mit einem Gehalt an p-Diisopropylbenzol-bis-perneodecanoat von 50 % (62 % der Theorie). Der Gehalt an Aktivsauerstoff beträgt 3,0 %.

Beispiel 2

In 384 g (2,0 Mol) 30 % Kalilauge werden unter Rühren 227 g (0,5 Mol) p-Bis-(hydroperoxy-isopropyl)-benzol (50 % wasserfeucht) gelöst und bei 20 °C 133 g (1,1 Mol) Pivaloylchlorid zugetropft. Bei 20 °C wird noch 30 Minuten nachgerührt. Der ausgefallene Feststoff wird abfiltriert und auf dem Filter mit Wasser neutral gewaschen. Man erhält 220 g eines wasserfeuchten Pulvers mit einem Gehalt an p-Diisopropylbenzol-bisperneopentanoat von 56 % (62 % der Theorie bezogen auf Dihydroperoxid). Der Gehalt an Aktivsauerstoff beträgt 4,5 %.

Beispiel 3

Das nach Beispiel 1 hergestellte p-Diisopropylbenzolbisperneodecanoat (p-DIPB-bis-PND), phlegmatisiert in Isododecan, wurde auf seine Polymerisationswirksamkeit bei der Polymerisation von Vinylchlorid geprüft. Es wurde bei Temperaturen von (a) 45 °C, (b) 50 °C und (c) 55 °C, und bei Polymerisationszeiten von (a) 2, (b) 4 und (c) 6 Stunden in einem Stahlautoklaven (Suspensionspolymerisation) gearbeitet.

Als Vergleich diente CUPND und ACSP.

Die erhaltenen Ergebnisse sind in der nachfolgenden Tabelle zusammengestellt (OP = organisches Peroxid).

## Tabelle

Ansatz: 130 g Aqua bidest +0,15 % PVA + 70 g Vinylchlorid + Peroxid

| T (°C) | Initiator | Dosierung: 100% OP mmol/70g VC | Dosierung: OP i.Lieferf. mg/70 g VC | Dosierung: OP i.Lf. (%) | Dosierung: 100% OP (%) | Umsatz (%) nach t (h): 2 | 4 | 6 |
|---|---|---|---|---|---|---|---|---|
| 45 | p-DIPB-bis PND 62 % in Isododecan | 0,060 | 51,8 | 0,07 | 0,05 | 11,7 | 33,4 | 50,1 |
| 45 | CUPND, 81 % in Isododecan | 0,120 | 45,7 | 0,06 | 0,05 | 10,6 | 25,3 | 47,4 |
| 45 | ACSP, 28 % in Phthalat | 0,120 | 92,3 | 0,13 | 0,04 | 12,1 | 22,3 | 47,0 |
| | | | | | | | | |
| 50 | p-DIPB-bis-PND 62 % in Isododecan | 0,060 | 51,8 | 0,07 | 0,05 | 21,4 | 46,4 | 66,0 |
| 50 | CUPND, 81 % in Isododecan | 0,119 | 45,3 | 0,06 | 0,05 | 19,0 | 43,6 | 61,7 |
| 50 | ACSP, 28 % in Phthalat | 0,120 | 94,7 | 0,14 | 0,04 | 21,9 | 51,7 | 62,9 |
| | | | | | | | | |
| 55 | p-DIPB-bis-PND 62 % in Isododecan | 0,060 | 51,8 | 0,07 | 0,05 | 31,4 | 58,1 | 69,9 |
| 55 | CUPND, 81 % in Isododecan | 0,120 | 45,7 | 0,06 | 0,05 | 27,9 | 57,0 | 67,3 |
| 55 | ACSP, 28 % in Phthalat | 0,120 | 89,8 | 0,13 | 0,04 | 35,3 | 56,4 | 62,7 |

**Patentansprüche**

1. Diisopropylbenzol-bisperneoalkanoate der allgemeinen Formel I

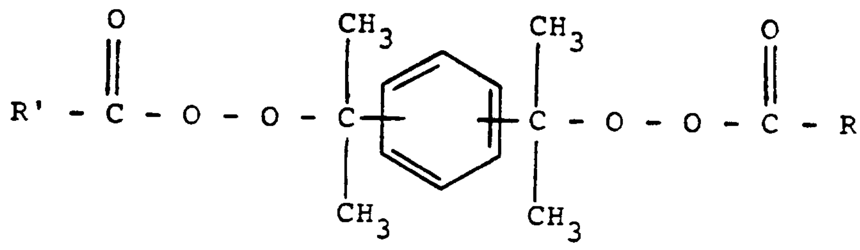

(I)

in der R und R' eine-$CR_1R_2R_3$-Gruppe bedeuten, worin $R_1$, $R_2$ und $R_3$ eine Alkylgruppe bedeutet, und $R_1$, $R_2$ und $R_3$ zusammen 3 bis 11 Kohlenstoffatome enthalten, R und R' verschieden oder gleich sind, und im Diisopropylbenzol-Rest die Isopropylgruppen in m- und/oder p-Stellung zueinander stehen.

2. Diisopropylbenzol-bisperneoalkanoate nach Anspruch 1,
**dadurch gekennzeichnet,**
daß die Reste $R_1$, $R_2$ und $R_3$ zusammen 5 bis 8 Kohlenstoffatome enthalten.

3. Diisopropylbenzol-bisperneoalkanoate nach Anspruch 1 oder 2,
**dadurch gekennzeichnet,**
daß R und R' gleich sind.

4. Diisopropylbenzol-bisperneodecanoat.

5. Verfahren zur Herstellung der Diisopropylbenzol-bisperneoalkanoate der allgemeinen Formel I nach einem der Ansprüche 1 bis 4,
**dadurch gekennzeichnet,**
daß man äquivalente Mengen von Bis-(hydroperoxyisopropyl)-benzol mit einer Verbindung RCOCl und/oder R'COCl, worin R und R' die im Anspruch 1 angegebene Bedeutung besitzen, in an sich bekannter Weise umsetzt.

6. Verwendung der Diisopropylbenzol-bisperneoalkanoate nach einem der Ansprüche 1 bis 4 als Initiatoren von Radikalkettenpolymerisationen oder -mischpolymerisationen.

7. Verwendung nach Anspruch 6 zur Polymerisation von Vinylchlorid.

8. Verwendung nach Anspruch 6 zur Polymerisation von Ethylen.

**Claims**

1. Diisopropylbenzene-bisperneoalkanoates of the general formula I

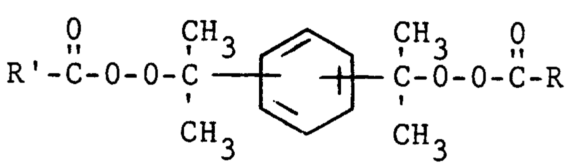

(I)

in which R and R' signify a -$CR_1R_2R_3$ group, wherein $R_1$, $R_2$ and $R_3$ signify an alkyl group, and $R_1$, $R_2$ and $R_3$ together contain 3 to 11 carbon atoms, R and R' are the same or different and, in the diisopropylbenzene radical, the isopropyl groups stand in the m- and/or p-position to one another.

2. Diisopropylbenzene-bisperneoalkanoates according to claim 1, characterised in that the radicals $R_1$, $R_2$ and $R_3$ together contain 5 to 8 carbon atoms.

3. Diisopropylbenzene-bisperneoalkanoates according to claim 1 or 2, characterised in that R and R' are the same.

4. Diisopropylbenzene-bisperneodecanoate.

5. Process for the preparation of the diisopropylbenzene-bisperneoalkanoates of the general formula I according to one of claims 1 to 4, characterised in that one reacts equivalent amounts of bis-(hydroperoxyisopropyl)-benzene with a compound RCOCl and/or R'COCl, wherein R and R' possess the meaning given in claim 1, in per se known way.

6. Use of the diisopropylbenzene-bisperneoalkanoates according to one of claims 1 to 4 as initiators of radical chain polymerisations or co-polymerisations.

7. Use according to claim 6 for the polymerisation of vinyl chloride.

8. Use according to claim 6 for the polymerisation of ethylene.

**Revendications**

1. Bisperneoalcanoates de diisopropylbenzène de formule générale I

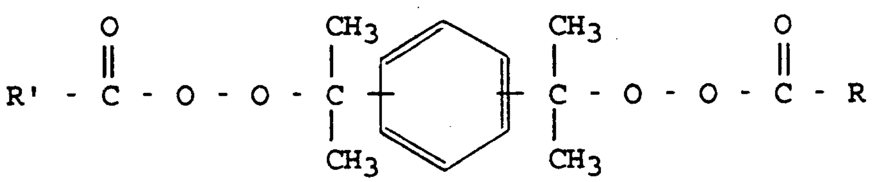

(I)

dans laquelle R et R' représentent un groupe $-CR_1R_2R_3$ dans lequel $R_1$, $R_2$ et $R_3$ représentent un groupe alkyle et $R_1$, $R_2$ et $R_3$ contiennent ensemble 3 à 11 atomes de carbone, R et R' sont différents ou identiques, et dans le reste diisopropylbenzène les groupes isopropyle sont en position m et/ou p l'un par rapport à l'autre.

2. Bispernéoalcanoates de diisopropylbenzène selon la revendication 1, caractérisés en ce que les restes $R_1$, $R_2$ et $R_3$ contiennent ensemble 5 à 8 atomes de carbone.

3. Bispernéoalcanoates de diisopropylbenzène selon la revendication 1 ou 2, caractérisés en ce que R et R' sont identiques.

4. Bispernéodécanoate de diisopropylbenzène.

5. Procédé de préparation des bispernéoalcanoates de diisopropylbenzène de formule générale I selon l'une des revendications 1 à 4, caractérisé en ce que l'on fait réagir de manière connue en soi des quantités équivalentes de bis-(hydroperoxy-isopropyl)-benzène avec un composé RCOCl et/ou R'COCl, dans lequel R et R' ont la signification indiquée dans la revendication 1.

6. Utilisation des bispernéoalcanoates de diisopropylbenzène selon l'une des revendications 1 à 4 comme amorceurs de polymérisations ou copolymérisations radicalaires.

7. Utilisation selon la revendication 6 pour la polymérisation du chlorure de vinyle.

8. Utilisation selon la revendication 6 pour la polymérisation de l'éthylène.